# NEUE EUROPÄISCHE PATENTSCHRIFT

(11) **EP 0 424 861 B2**
(45) Veröffentlichungstag und Bekanntmachung des Hinweises auf die Entscheidung über den Einspruch: **22.04.1998**
(45) Hinweis auf die Patenterteilung: 14.09.1994
(21) Anmeldenummer: 90120270.5
(22) Anmeldetag: 23.10.1990
(51) Int. Cl.: C07C 69/63, C07C 67/08, C07C 67/54

(54) **Verfahren zur kontinuierlichen Herstellung von Estern niederer aliphatischer Carbonsäuren mit niederen Alkoholen**
Process for the continuous preparation of esters of lower carboxylic acid with lower alcohols
Procédé de préparation continue d'esters d'acides carboxyliques inférieurs et d'alcools inférieurs

(30) Priorität: 25.10.1989 DE 3935470
(43) Veröffentlichungstag der Anmeldung: 02.05.1991
(73) Patentinhaber: Clariant GmbH, 65929 Frankfurt am Main (DE)
(72) Erfinder: Miltenberger, Karlheinz, Dr., W-8906 Gersthofen (DE); Schmidt, Manfred, W-8870 Günzburg-Deffingen (DE); Petz, Karl, Dr., W-8902 Neusäss (DE)

(56) Entgegenhaltungen:
- EP-A- 0 315 096
- DD-A- 32 019
- DD-A- 97 416
- JP-A-57 156 439
- RO-A- 83 054
- D'Ans-Lax, Taschenbuch für Chemiker und Physiker, Bd. II, S. 2-267 bis 2-268
- Kirk-Othmer, Encyclopedia of Chemical Technology, 3. Ausgabe, Bd. 1, S. 341-342
- Römpps Chemie-Lexikon, 8. Auflage, 1979, Franckh'sche Verlagshandlung Stuttgart, Bd. 1, S. 112

## Beschreibung

Die Erfindung bezieht sich auf ein kontinuierliches Verfahren zur Herstellung von Estern niederer aliphatischer Carbonsäuren mit niederen Alkoholen, insbesondere der Monochloressigsäure mit C₁-C₄-Alkoholen.

Bekannt ist ein Verfahren zur Herstelltug von Estern der Monochloressigsäure mit C₁-C₄-Alkoholen durch Veresterung der freien Säure mit den Alkoholen in Gegenwart von Veresterungskatalysatoren, bei dem der Alkohol der katalysatorhaltigen Schmelze der Monochloressigsäure unter Entfernung des Reaktionswassers mit solcher Geschwindigkeit zugegeben wird, daß das sich bildende Reaktionswasser zusammen mit Anteilen des entstandenen Chloressigsäureesters als praktisch alkoholfreies binäres Azeotrop abdestilliert wird (vgl. EP 315 096). Die Hauptmenge des Esters wird als Sumpfprodukt gewonnen und muß zur Entfernung unumgesetzter Säure und des Katalysators mit wäßriger Bicarbonatlösung gewaschen werden. Dabei fällt salzhaltiges Abwasser an.

Die Aufgabe bestand darin, ein kontinuierliches Veresterungsverfahren zu finden, bei welchem keine salzhaltigen Abwässer anfallen.

Es wurde gefunden, daß die Aufgabe gelöst werden kann, wenn man den entstandenen Ester kontinuierlich abdestilliert und mit Wasser wäscht.

Die Erfindung betrifft somit ein Verfahren zur Herstellung eines Esters einer niederen aliphatischen C₂C₆-Carbonsäure mit einem niederen aliphatischen C₁-C₄-Alkohol durch Reaktion der Carbonsäure mit dem Alkohol bei erhöhter Temperatur in Gegenwart eines Katalysators unter Abtrennung des Reaktionswassers durch Destillation, dadurch gekennzeichnet daß die Säure in flüssiger Form zusammen mit dem Katalysator vorgelegt, der Alkohol in die Säure eingeleitet, der entstandene Ester zusammen mit dem Reaktionswasser azeotrop abdestilliert, nach Abscheidung des Reaktionswassers mit Wasser im Gegenstrom gewaschen und danach durch Abdestillieren eines kleinen Teils des Esters von Restmengen an Wasser und Alkohol befreit wird.

Das erfindungsgemäße Verfahren wird in der folgenden Weise durchgeführt.

Die zu verestemde Säure wird in einem Reaktionsgefäß in flüssiger Form vorgelegt und der Alkohol wird am Boden des Reaktionsgefäßes in die Säure eingeleitet, vorzugsweise in Dampfform. Beim Einleiten von Dampf wird zweckmäßigerweise eine Düse verwendet, beim Einleiten des flüssigen Alkohols genügt ein einfaches Rohr. Dabei wird durch den verdampfenden Alkohol eine Zwangsumwälzung des Ansatzes erreicht. Der Katalysator befindet sich in der vorgelegten Säure. Die Reaktionsprodukte Ester und Wasser destillieren ab, ihre Dämpfe werden durch eine kurze Rektifikationskolonne mit Rücklaufteiler geführt. Durch Wahl eines geeigneten Rücklaufverhältnisses, welches von der Art des Esters und der Reaktionstemperatur abhängt, läßt sich die mitgerissene Menge an Säure und Alkohol auf ein Minimum einstellen. Für Ester der Monochloressigsäure wurde gefunden

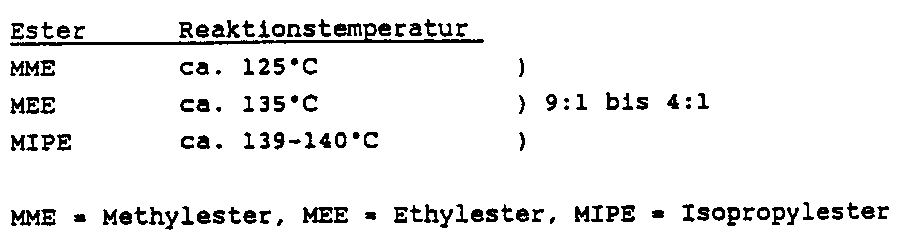

Erhöht man die Reaktionstemperatur, so steigt der Anteil der Monochloressigsäure im Ester an und der Alkoholanteil geht zurück. Bei Temperaturerniedrigung steigt der Alkoholanteil und der Säureanteil geht zurück. Im gleichen Maße, wie der entstandene Ester zusammen mit dem Reaktionswasser abdestilliert. werden Saure und Alkohol nachgeführt. Das Gemisch gelangt sodann in einen Trennkühler, wo eine spontane Trennung in eine wäßrige Phase und eine Esterphase erfolgt. Die Wasserphase wird zur Aufbereitung gerbracht, wo ihr die Restmengen Ester, Säure und Alkohol entzogen werden. Die Esterphase aus dem Trennkühler wird in eine Waschkolonne überführt und dort im Gegenstrom mit Wasser gewaschen. Zur Intensivierung des Wascheffektes ist die Kolonne vorzugsweise mit einer Pulsationspumpe ausgerüstet. Der nunmehr säurefreie Ester wird von Resten von Wasser und Alkohol befreit. Dafür ist ein Fallfilmverdampfer geeignet, welcher unter leichtem Vakuum betrieben wird. Möglich ist jedoch auch ein chargenweiser Betrieb. Dazu gelangt der Ester über ein Püffergefäß chargenweise in eine Destillierblase, wo unter leichtem Vakuum Wasser, Alkohol und ein kleiner Anteil Ester (maximal 10 %) abdestilliert (abgetoppt) werden. Der die Destillierblase verlassende Ester ist frei von Nebenprodukten. Das Destillat wird getrennt, die Esterphase zurückgeführt in die Waschkolonne und die Wasserphase zur Aufbereitung gegeben. In der Aufbereitung zurückgewonnene Säure und zurückgewonnener Alkohol werden wieder in die Veresterungsstufe eingeführt. Das zurückbleibende Wasser kann in den Kanal abgelassen werden.

Nach dem erfindungsgemäßen Verfahren werden niedere aliphatische Carbonsäuren mit 2 bis 6 C-Atomen verestert. Vorzugsweise werden die niederen Halogencarbonsäuren, wie beispielsweise Chloressigsäuren oder Chlorpropionsäuren, insbesondere Monochloressigsäure, eingesetzt.

Die zu verwendenden Alkohole sind aliphatische C₁-C₄-Alkohole, beispielsweise Methanol, Ethanol, Isopropanol.

Säure und Alkohol werden derart ausgewählt, daß der entstehende Ester bei Temperaturen um 20°C noch flüssig ist und vorzugsweise eine Dichte größer als Wasser besitzt.

Als Katalysator wird ein bekannter Veresterungskatalysator, wie beispielsweise AlCl₃, konzentrierte Schwefelsäure, Methansulfonsäure, Ethansulfonsäure, p-Toluolsulfonsäure verwendet, vorzugsweise Schwefelsäure oder Methansulfonsäure. Die Einsatzmenge beträgt 0,1 bis 1 % der im Reaktionsgefäß vorhandenen Säuremenge.

Die Reaktionstemperatur beträgt 100 bis 150°C, vorzugsweise 125 bis 140°C.

Das erfindungsgemäße Verfahren liefert einen reinen Ester in hoher Ausbeute. Salzhaltige Abwässer fallen in der Regel nicht an.

Die nachfolgenden Beispiele sollen die Erfindung erläutern.

### Beispiel 1

In einer Anlage gemäß der Figur wurde Monochloressigsäuremethylester (MME) hergestellt.

Der verwendete Reaktor (1) bestand aus einem mantelbeheizten Emaille-Unterteil (1a), einem mit Raschigringen gefüllten Kolonnenschuß (1b), einem Rücklaufteiler (1c) und zwei Kühlschüssen (1d) und (1e) aus Glas.
Im Reaktorunterteil befanden sich ca. 650 kg geschmolzene Monochloressigsäure (= ca. 70 % des Volumens) und 5 l konzentrierte Schwefelsäure bei einer Temperatur von 125°C. Stündlich wurden aus dem Vorratsgefäß (3) über den Wärmetauscher (4) 155 kg Methanol dampfförmig mit einer Temperatur von 108°C in den Reaktor eingeführt. Bedingt durch das Abdestillieren von Methylester und Wasser strömten aus dem beheizten Vorratsgefäß (2) 400 kg/h Monochloressigsäure in den Reaktor. Das Rücklaufverhältnis in der Kolonne (1b)-(1e) wurde über eine Taktregelung gesteuert und auf 1:7,5 (Abnahme) eingeregelt. Dabei bildete sich im Reaktor ein Gleichgewicht zwischen den Reaktanden.

Nach Einsetzen der Destillation fiel im Trennkühler (5) ein Kondensat an, das sich spontan in zwei Phasen trennte. Die schwerere, untere Phase war die Esterphase, insgesamt 489 kg/h. Sie hatte folgende Zusammensetzung:

| | |
|---|---|
| MME | 91,3 % |
| Methanol | 2,9 % |
| MS | 1,3 % |
| Wasser | 4,5 % |
| (MS = Monochloressigsäure) | |

Als obere Phase fielen stündlich 66 kg/h Reaktionswasser an:

| | |
|---|---|
| Wasser | 83,7 % |
| Methanol | 10,0 % |
| MS | 1,3 % |
| MME | 5,0 % |

Dieses Reaktionswasser wurde zur Aufbereitung gegeben. Der rohe Ester wurde zur Entfernung der Monochloressigsäure in der Waschkolonne (6) im Gegenstrom mit 795 dm³/h Wasser gewaschen, wobei der Inhalt der Kolonne zur Erhöhung der Waschwirkung durch eine Pulsationspumpe (7) in pulsierende Bewegung gebracht wurde.
423 kg/h Ester mit der Zusammensetzung

| | |
|---|---|
| MME | 98, 2 % |
| Methanol | 0,2 % |
| Wasser | 1,6 % |

und 860 kg/h Waschwasser mit der Zusammensetzung

| | |
|---|---|
| Wasser | 94,5 % |
| MME | 3,3 % |
| Methanol | 1,5 % |
| MS | 0,7 % |

verließen die Waschkolonne (6). Der Ester wurde zunächst in das Zwischengefäß (8) überführt. Aus diesem Zwischengefäß (8) wurden bei Bedarf jeweils 4500 kg Ester in die Destillierblase (9) eingefüllt und etwa 10 % des Esters in ca. 4 Stunden bei 80 bis 85°C und 0,15 bis 0,20 bar abdestilliert. Der in der Blase (9) verbliebene Ester war säure- und methanolfrei. Der über die kleine Kolonne (10) abdestillierte und in der Vorlage (11) abgetrennte Anteil des Esters enthielt

| | |
|---|---|
| MME | 81 % |
| Methanol | 1,6 % |
| Wasser | 17,5 % |

und wurde über die Leitung (12) der Waschkolonne zugeführt.

Die Ausbeute betrug ca. 100 kg/h reinen Monochloressigsäuremethylester, das bedeutet 97 bis 98 %, bezogen auf die eingesetzte Säure und 88 bis 89 %, bezogen auf eingesetztes Methanol.

### Beispiel 2

In der gleichen Anlage wie in Beispiel 1 wurde Monochloressigsäureethylester (MEE) hergestellt mit folgenden Daten:

| | |
|---|---|
| Reaktionstemperatur | 135°C |
| Monochloressigsäure | 315 kg/h |
| Ethanol | 186 kg/h |
| Katalysator | H₂SO₄, ca. 1 % |
| Rücklaufverhältnis | 1:5 |

| Rohester aus Trennkühler (5) 436 kg/h | |
|---|---|
| MEE | 90,1 % |
| Ethanol | 5,1 % |
| MS | 1,9 % |
| Wasser | 2,9 % |

| Reaktionswasser 65 kg/h | |
|---|---|
| Wasser | 91,4 % |
| Ethanol | 5,3 % |
| MS | 2,0 % |
| MEE | 1,3 % |

| Ester aus Waschkolonne (6) 377 kg/h | |
|---|---|
| MEE | 98,3 % |
| Ethanol | 0,6 % |
| Wasser | 1,1 % |

| Waschwasser aus Waschkolonne (6) 818 kg/h | |
|---|---|
| Wasser | 94,4 % |
| Ethanol | 2,4 % |
| MS | 1,0 % |
| MEE | 2,2 % |

Der Ester wurde bei Bedarf chargenweise bei 90 bis 95°C und 0,15 bis 0,20 bar getoppt. Ausbeute , säure-und ethanolfrei, 96 bis 97 %, bezogen auf eingesetzte Säure, und 81 bis 82 %, bezogen auf eingesetztes Ethanol. Wassergehalt maximal 0,01 %.

### Beispiel 3

In der Anlage wie in Beispiel 1 wurde Monochloressigsäureisopropylester hergestellt.

| | |
|---|---|
| Reaktionstemperatur | 135-140°C |
| Monochloressigsäure | 62 kg/h |
| Isopropanol | 55 kg/h |
| Katalysator | Schwefelsäure, 0,1 % |

| Wasserdampf durch Leitung (13) zur Verringerung von Nebenreaktionen, 3 bar 20 kg/h | |
|---|---|
| Rücklaufverhältnis | 1:5 (Abnahme) |

| Rohester aus Trennkühler (5) 100 kg/h | |
|---|---|
| MIPE | 83,0 % |
| Isopropanol | 7,2 % |
| MS | 1,8 % |
| Wasser | 8,0 % |

| Reaktionswasser aus Trennkühler (5) 33 kg/h | |
|---|---|
| Wasser | 93,5 % |
| Isopropanol | 3,6 % |
| MS | 2,4 % |
| MIPE | 0,5 % |

| Ester aus Waschkolonne (6) 92 kg/h | |
|---|---|
| MIPE | 98,9 % |
| Isopropanol | 0,2 % |
| Wasser | 0,9 % |

| Waschwasser aus Waschkolonne 215 kg/h | |
|---|---|
| Wasser | 95,1 % |
| Isopropanol | 3,2 % |
| MS | 0,8 % |
| MIPE | 0,9 % |

Der nach der Trocknung anfallende Ester war frei von Säure und Isopropanol:

| | |
|---|---|
| MIPE | 99,7 % |
| Diisopropylether | 0,2 % |
| Wasser | 0,1 % |

Ausbeute 90 bis 91 %, bezogen auf eingesetzte Säure und 72 bis 75 %, bezogen auf eingesetztes Isopropanol.

Bei der Veresterung von Monochloressigsäure mit Isopropanol ist der Anteil mitgerissener Monochloressigsäure wegen der höheren Veresterungstemperatur deutlich erhöht. Außerdem machen sich Nebenreaktionen wie Bildung von Diisopropylether und Propen durch Wasserabspaltung aus Isopropanol bemerkbar. Diese Nebenreaktionen können durch Zugabe von Wasserdampf zurückgedrängt werden.

### Beispiel 4

In einer Laborapparatur, bestehend aus einem 500 cm³-Vierhalskolben mit Rührer, Einleitrohr für Säure-Methanol-Gemisch, Schliffthermometer und einer aufgesetzten versilberten Vakuumkolonne von 1 m Länge und 3 cm Durchmesser, Intensivkühler und Auffangbehälter, wurden ca. 1200 g/h einer Mischung von 9044 g 1-Chlorpropionsäure 96%ig (= 80 mol) und 2715 g Methanol (= 85 mol) bei einer Temperatur von 100 bis 105°C verestert. Dazu wurde die gesamte 1-Chlorpropionsäure außerhalb der Apparatur in Methanol aufgelöst. Danach wurden in dem 500-cm³-Vierhalskolben ca. 250 cm³ der 1-Chlorpropionsäure/Methanol-Lösung ohne Katalysator vorgelegt. Das Ölbad wurde aufgeheizt, wobei über die Kolonne Ester/Wasser-Gemisch bei 100 - 105°C und ein Rücklaufverhältnis 1:5 (Abnahme) abdestillierte. Über das Einleitrohr wurde das restliche Reaktionsgemisch kontinuierlich auf den Kolbengrund zudosiert. Die Zudosierung erfolgte in demselben Maße, wie das Ester/Wasser-Gemisch abdestillierte (ca. 1 l/h).

Rohester und Reaktionswasser wurden getrennt aufgefangen und hatten folgende Zusammensetzung:

| CG-Analyse | Roh-CPE | Reaktionswasser |
|---|---|---|
| ? | 0,02 % | 0,02 % |
| Methanol | 3,33 % | 8,90 % |
| ? | 0,08% | - |
| Wasser | 0,90 % | 89,24 % |
| CPE | 92,10 % | Spur |
| ? | 0,12% | - |
| DCPE | 3,05 % | - |
| 1-Chlorpropionsäure | 0,40 % | 1,90 % |

Die Ausbeute an Rohester betrug ca. 92 %.

Der Rohester wurde sodann in einer Glassäule von 1 m Länge und einem Durchmesser von 30 mm, gefüllt mit Raschigringen (⌀ 6 mm), in einer Menge von 0,35 kg/h im Gegenstrom mit der vierfachen Menge Wasser gewaschen. Da die Mischung der Reaktanten offenbar nicht optimal war, ging der Gehalt an 1-Chlorpropionsäure von 0,40 % nur auf 0,14 zurück. Aus diesem Grund wurde eine Wäsche mit einer Bicarbonatlösung angeschlossen. Danach hatte der Ester die folgende Zusammensetzung:

| | |
|---|---|
| ? | 0,22 % |
| Methanol | 1,93 % |
| Wasser | 0,50 % |
| CPE | 93,78 % |
| DCPE | 3,55 % |
| ? | 0,08% |

Je 600 g dieses Esters wurden in einem 1 dm³-Rundkolben bei 60°C und einem Druck von 0,13 bar einer Kolonnendestillation unterworfen. Nach Abdestillieren von 4 bis 5 % hatte der Ester die Zusammensetzung

| | |
|---|---|
| Methanol | 0,11 % |
| Wasser | 0,04 % |
| CPE | 95,90 % |
| DCPE | 3,90 % |

und war für die Weiterverarbeitung hinreichend rein.

## Patentansprüche

1. Verfahren zur Herstellung eines Esters einer niederen aliphatischen C₂-C₆-Carbonsäure mit einem niederen aliphatischen C₁-C₄-Alkohol durch Reaktion der Carbonsäure mit dem Alkohol bei erhöhter Temperatur in Gegenwart eines Katalysators unter Abtrennung des Reaktionswassers durch Destillation, dadurch gekennzeichnet, daß die Säure in flüssiger Form zusammen mit dem Katalysator vorgelegt, der Alkohol in die Säure eingeleitet, der entstandene Ester zusammen mit dem Reaktionswasser azeotrop abdestilliert, nach Abscheidung des Reaktionswassers mit Wasser im Gegenstrom gewaschen und danach durch Abdestillieren eines kleinen Teils des Esters von Restmengen an Wasser und Alkohol befreit wird.

2. Verfahren nach nach Anspruch 1, dadurch gekennzeichnet, daß eine aliphatische C₂-C₆-Halogencarbonsäure verestert wird.

3. Verfahren nach Anspruch 2, dadurch gekennzeichnet, daß Monochloressigsäure verestert wird.

## Claims

1. A process for the preparation of an ester of a lower aliphatic C₂-C₆-carboxylic acid with a lower aliphatic C₁-C₄-alcohol by reacting the carboxylic acid with the alcohol at an elevated temperature in the presence of a catalyst with removal of the water of reaction by distillation, which comprises initially taking the acid, in liquid form, together with the catalyst, introducing the alcohol into the acid, removing the ester formed together with the water of reaction by azeotropic distillation, washing the ester countercurrent with water after separating off the water of reaction and then freeing the ester from residual amounts of water and alcohol by distilling off a small fraction of the ester.

2. The process as claimed in claim 1, wherein an aliphatic C₂-C₆-halogenocarboxylic acid is esterified.

3. The process as claimed in claim 2, wherein monochloroacetic acid is esterified.

## Revendications

1. Procédé pour la préparation d'un ester d'un acide carboxylique en C₂-C₆ aliphatique inférieur avec un alcool en C₁-C₄ aliphatique inférieur, par réaction de l'acide carboxylique avec l'alcool, à température élevée, en présence d'un catalyseur, en séparant par distillation l'eau de réaction, caractérisé en ce que l'on place préalablement ensemble l'acide sous forme liquide avec le catalyseur, on introduit l'alcool dans l'acide, on sépare l'ester formé par distillation azéotropique conjointement avec l'eau de réaction, après séparation de l'eau de réaction, on lave à contre-courant avec de l'eau, puis par distillation d'une petite portion d'ester, on le débarasse des quantités résiduelles d'eau et d'alcool.

2. Procédé selon la revendication 1, caractérisé en ce qu'on estérifie un acide halogénocarboxylique en C₂-C₆ aliphatique.

3. Procédé selon la revendication 2, caractérisé en ce qu'on estérifie l'acide monochloracétique.
